# EUROPÄISCHE PATENTSCHRIFT

(11) **EP 4 355 263 B1**
(45) Veröffentlichungstag und Bekanntmachung des Hinweises auf die Patenterteilung: **27.11.2024**
(21) Anmeldenummer: 22732970.3
(22) Anmeldetag: 02.06.2022
(51) Int. Cl.: A61F 2/16

(54) **INJEKTOR MIT EINER EINE GEWELLTE WAND AUFWEISENDEN INJEKTORSPITZE**
INJECTOR WITH AN INJECTOR TIP HAVING A CORRUGATED WALL
INJECTEUR AVEC UNE POINTE D'INJECTEUR AYANT UNE PAROI ONDULÉE

(30) Priorität: 16.06.2021 DE 102021115562
(43) Veröffentlichungstag der Anmeldung: 24.04.2024
(73) Patentinhaber: Carl Zeiss Meditec AG, 07745 Jena (DE)
(72) Erfinder: MÜLLER, Marco, 10437 Berlin (DE)
(74) Vertreter: PATERIS Patentanwälte PartmbB
(86) Internationale Anmeldenummer: PCT/EP2022/065124
(87) Internationale Veröffentlichungsnummer: WO 2022/263200

(56) Entgegenhaltungen:
- EP-A1- 1 287 792
- WO-A2-98/37830
- US-A1- 2007 005 135

## Beschreibung

Die Erfindung betrifft einen Injektor für ein Einführen einer Intraokularlinse in den Kapselsack eines Auges. US 2007/0005135 A1 offenbart eine Einführungsapparatur zum Implantieren von Intraokularlinsen durch kleinere Einschnitte. EP 1 287 792 A1 offenbart eine Einführungsvorrichtung für eine verformbare Intraokularlinse. WO 98/37830 A2 offenbart eine Implantationsvorrichtung mit einer verformbaren Düsenspitze zum Implantieren einer verformbaren Intraokularlinse.

Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack des Auges abgesaugt. Danach wird eine Intraokularlinse mittels des Injektors in das Auge eingesetzt. Die Intraokularlinse wird in dem Injektor gefaltet, so dass sie durch die Spitze passt. Die Spitze wird durch den Schnitt in den Kapselsack eingeführt und die gefaltete Intraokularlinse wird mittels des Injektors durch die Spitze in den Kapselsack geschoben, in dem die Intraokularlinse sich entfaltet und somit die ursprüngliche Linse ersetzt.

Um den Schnitt in der Hornhaut möglichst klein ausführen zu können, ist es erforderlich, dass die Spitze des Injektors möglichst klein ausgeführt ist. Dies führt dazu, dass die Intraokularlinse stark gequetscht wird, wenn die Intraokularlinse in der Spitze angeordnet ist. Dies führt zu einer hohen mechanischen Belastung der Spitze, wobei die hohe mechanische Belastung zu einer Beschädigung der Spitze führen kann. Beispielsweise können Teile der Spitze irreversibel nach außen verformt werden, was im Extremfall dazu führen kann, dass die Spitze eine Erweiterung des Schnitts und/oder eine Beschädigung des Auges verursachen kann, wenn die Spitze aus dem Auge herausgezogen wird.

Aufgabe der Erfindung ist es daher, einen Injektor für ein Einführen einer Intraokularlinse in den Kapselsack eines Auges zu schaffen, wobei eine Spitze des Injektors klein ausgeführt werden kann, ohne dass die Gefahr einer Beschädigung der Spitze auftritt.

Der erfindungsgemäße Injektor für ein Einführen einer Intraokularlinse in den Kapselsack eines Auges weist eine Injektorachse, eine Injektorspitze, die eine Öffnung und eine die Öffnung begrenzende Wand aufweist, und einen Kolben auf, der in Richtung der Injektorachse längsverlagerbar in dem Injektor gelagert ist und eingerichtet ist, die Intraokularlinse via die Öffnung aus dem Injektor zu befördern, wobei die Wand in einem ersten Abschnitt, der von dem Ende der Injektorspitze, an dem die Öffnung angeordnet ist, ausgeht und sich in Richtung der Injektorachse erstreckt, einen Elastizitätsmodul unter einer Zugbelastung in einem Bereich von 10 N/mm² bis 500 N/mm² hat und gewellt ausgeführt ist, so dass die Wand in einer Querschnittsebene, deren Normale parallel zu der Injektorachse angeordnet ist, eine Mehrzahl von Bergen und eine Mehrzahl von Tälern aufweist, wobei die Berge und die Täler in Umfangsrichtung bezüglich der Injektorachse abwechselnd angeordnet sind.

Dadurch, dass der Elastizitätsmodul in einem Bereich von 10 N/mm² bis 500 N/mm², bevorzugt zwischen 10 N/mm² und 250 N/mm² und besonders bevorzugt zwischen 10 N/mm² und 100 N/mm², liegt, kann die Wand elastisch gedehnt werden, wenn die Intraokularlinse in die Injektorspitze verlagert wird. Indem die Wand gewellt ausgeführt ist, können die Täler und optional auch die Berge in Radialrichtung bezüglich der Injektorachse nach außen verdrängt werden, wenn die Intraokularlinse in die Injektorspitze verlagert wird, wodurch sich der Querschnitt der Injektorspitze vergrößert. Dadurch kann die Injektorspitze in ihrem Zustand, bevor die Intraokularlinse in die Injektorspitze verlagert wird, kleiner als bei einer herkömmlichen Injektorspitze ausgeführt werden, bei der die Wand einen höheren Elastizitätsmodul als 500 N/mm² und nicht gewellt ausgeführt ist. Die von der gequetschten Intraokularlinse auf die Wand des ersten Abschnittes ausgeübte Druckkraft ist wegen der relativ hohen Elastizität des Werkstoffes des ersten Abschnittes geringer als bei einem Injektor, dessen erster Abschnitt aus einem Werkstoff mit einem Elastizitätsmodul unter der Zugbelastung von größer als 500 N/mm² ist. Dadurch ist eine Gefahr einer Beschädigung der Injektorspitze gering. Es ist denkbar, dass sich die Wand derart verformt, dass ein Innenraum in der Injektorspitze ein größeres Volumen als bei der herkömmlichen Injektorspitze annimmt. Dadurch wird die Intraokularlinse weniger als bei der herkömmlichen Injektorspitze gequetscht, wodurch auch die Gefahr einer Beschädigung der Intraokularlinse gering ist. Wenn die Intraokularlinse den Injektor via die Öffnung verlassen hat, nimmt die Wand wieder die Form an, die die Wand hatte, bevor die Intraokularlinse in die Injektorspitze verlagert wurde. Dadurch ist auch eine Gefahr einer Beschädigung der Hornhaut des Auges und/oder anderer Teile des Auges gering, wenn die Injektorspitze wieder aus dem Auge entfernt wird.

Es ist bevorzugt, dass die Injektorspitze einen entspannten Zustand, in dem die Intraokularlinse nicht in der Injektorspitze angeordnet ist, einen teilweise gespannten Zustand, in dem die Wand in dem ersten Abschnitt durch ein Einführen der Intraokularlinse in die Injektorspitze unter eine mechanische Zugspannung gesetzt ist, und einen maximal gespannten Zustand aufweist, in dem die Wand durch das Einführen der Intraokularlinse maximal gespannt ist, wobei die Öffnung in dem maximal gespannten Zustand größer als in dem entspannten Zustand ist. Dadurch kann die Öffnung, wie sie in dem entspannten Zustand vorliegt, kleiner als bei der herkömmlichen Injektorspitze ausgeführt werden, bei der die Injektorspitze nicht den Elastizitätsmodul im Bereich von 10 N/mm² bis 500 N/mm² hat und nicht gewellt ausgeführt ist.

In dem ersten Abschnitt und in dem entspannten Zustand verkleinert die Injektorspitze bevorzugt in Richtung zu der Öffnung hin kontinuierlich ihren Querschnitt, dessen Normale parallel zu der Injektorachse angeordnet ist. Dadurch ist der Querschnitt im Bereich der Öffnung kleiner als der verbliebene Teil des ersten Abschnitts, wodurch die Spitze besonders einfach in einen Schnitt in der Hornhaut eingefügt werden kann. Unter dem Querschnitt kann der Querschnitt an der Außenseite der Wand und/oder an der Innenseite der Wand gemeint sein.

Es ist bevorzugt, dass in dem ersten Abschnitt und in dem maximal gespannten Zustand die Injektorspitze in Richtung zu der Öffnung hin ihren Querschnitt, dessen Normale parallel zu der Injektorachse angeordnet ist, kontinuierlich vergrößert oder gleich groß belässt. In dem Fall, dass sich der Querschnitt kontinuierlich vergrößert, wird erreicht, dass eine maximale Quetschung der Intraokularlinse nicht unmittelbar an der Öffnung sondern entfernt von der Öffnung vorliegt. Wird die Intraokularlinse bei ihrer maximalen Quetschung verlagert, kann dies dazu führen, dass die Intraokularlinse sich unkontrolliert und mit einer hohen Geschwindigkeit bewegt. Indem der Ort der maximalen Quetschung fernab der Öffnung angeordnet ist, kann vermieden werden, dass die Intraokularlinse unkontrolliert und mit der hohen Geschwindigkeit die Injektorspitze verlässt. Unter dem Querschnitt kann der Querschnitt an der Außenseite der Wand und/oder an der Innenseite der Wand gemeint sein.

In dem ersten Abschnitt und in dem maximal gespannten Zustand hat die Wand bevorzugt an deren Außenseite und/oder deren Innenseite in der Querschnittsebene eine kreisförmige, elliptische und/oder ovale Form.

Die Wand hat bevorzugt in Umfangsrichtung bezüglich der Injektorachse und im Querschnitt eine konstante Dicke. Dies gilt für den entspannten Zustand, den teilweise gespannten Zustand und den maximal gespannten Zustand. Besonders bevorzugt ist die Dicke der Wand in dem gesamten ersten Abschnitt konstant. Es ist zudem bevorzugt, dass die Öffnung vollständig in einer Öffnungsebene liegt, die parallel zu der Querschnittsebene angeordnet sein kann oder deren Normale mit der Normalen der Querschnittsebene einen von 0° verschiedenen Winkel einschließen kann, und dass die Wand in dem ersten Abschnitt bis auf die Öffnung keine weitere Aussparung aufweist.

Die Wand weist bevorzugt einen zweiten Abschnitt auf, der sich in Richtung der Injektorachse erstreckt und in dem die Wand einen Elastizitätsmodul unter der Zugbelastung von höher als 900 N/mm², insbesondere höher oder gleich 1,8*10³ N/mm², hat. Die in dem zweiten Abschnitt vorgesehene Wand ist dazu vorgesehen, die Hornhaut zu kontaktieren, wenn die Intraokularlinse in die Injektorspitze verlagert wird, wodurch vermieden wird, dass dabei die Hornhaut verformt wird. Ein Übergang des Elastizitätsmoduls von dem ersten Abschnitt zu dem zweiten Abschnitt kann kontinuierlich erfolgen, beispielsweise entlang einer Strecke von mehr als 0,5 mm, mehr als 1 mm oder mehr als 2 mm.

Um zu erreichen, dass die Wand in dem zweiten Abschnitt einen höheren Widerstand unter der Zugbelastung hat, kann die Dicke der Wand in dem zweiten Abschnitt größer als in dem ersten Abschnitt sein. Alternativ oder zusätzlich ist denkbar, dass der zweite Abschnitt ein Verstärkungsstück aufweist, das in der Wand angeordnet ist. Das Verstärkungsstück ist bevorzugt eine Helix, ein zylindermantelförmiges Teil und/oder ein kegelmantelförmiges Teil oder weist bevorzugt die Helix, das zylindermantelförmige Teil und/oder das kegelmantelförmige Teil auf. Dabei ist besonders bevorzugt, dass das Verstärkungsstück ein Metall oder eine Legierung aufweist oder aus dem Metall oder der Legierung besteht.

Die Dicke der Wand kann in dem ersten Abschnitt gleich der Dicke der Wand in dem zweiten Abschnitt sein.

Es ist bevorzugt, dass die Wand jeweils von vier bis zwölf der Berge und der Täler aufweist, insbesondere jeweils von sechs bis zehn der Berge und der Täler oder jeweils acht der Berge und der Täler aufweist.

Der Injektor weist bevorzugt die Intraokularlinse auf.

Im Folgenden wird anhand der beigefügten schematischen Zeichnungen die Erfindung näher erläutert. Es zeigen
Figur 1 eine perspektivische Ansicht eines Injektors in einem entspannten Zustand schräg von vorne,
Figur 2 eine Seitenansicht des Injektors in dem entspannten Zustand,
Figur 3 die Ansicht des Injektors gemäß Figur 1 in einem vollständig gespannten Zustand des Injektors,
Figur 4 die Ansicht des Injektors gemäß Figur 2 in dem vollständig gespannten Zustand,
Figur 5 einen Längsschnitt durch eine bevorzugte Ausführungsform des Injektors,
Figur 6 einen Querschnitt entlang der in Figur 2 eingezeichneten Ebene A-A durch eine Injektorspitze des Injektors und
Figur 7 eine schematische Darstellung eines Längsschnittes des in Figur 1 gezeigten Injektors.

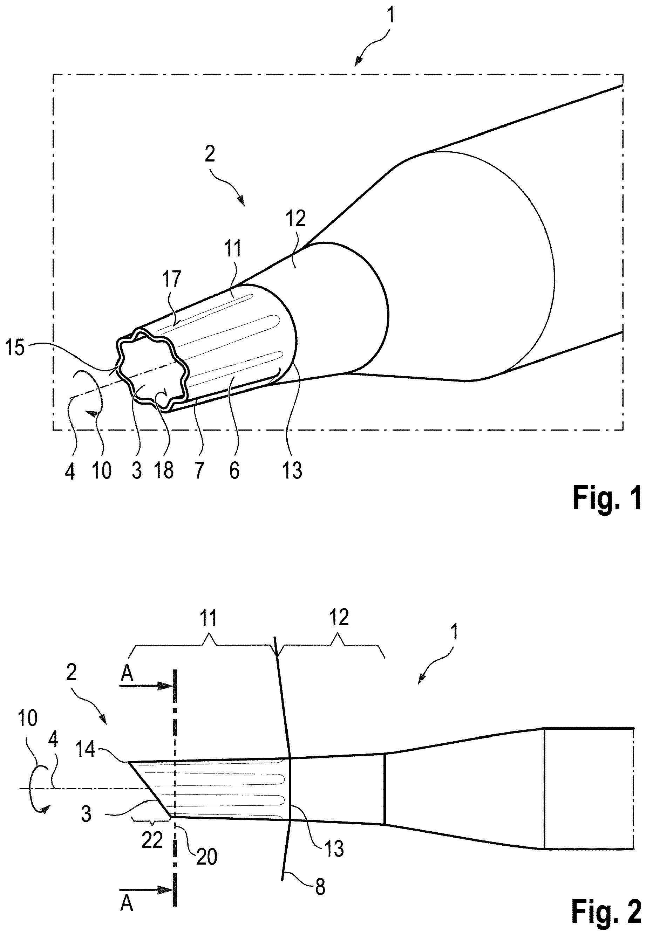

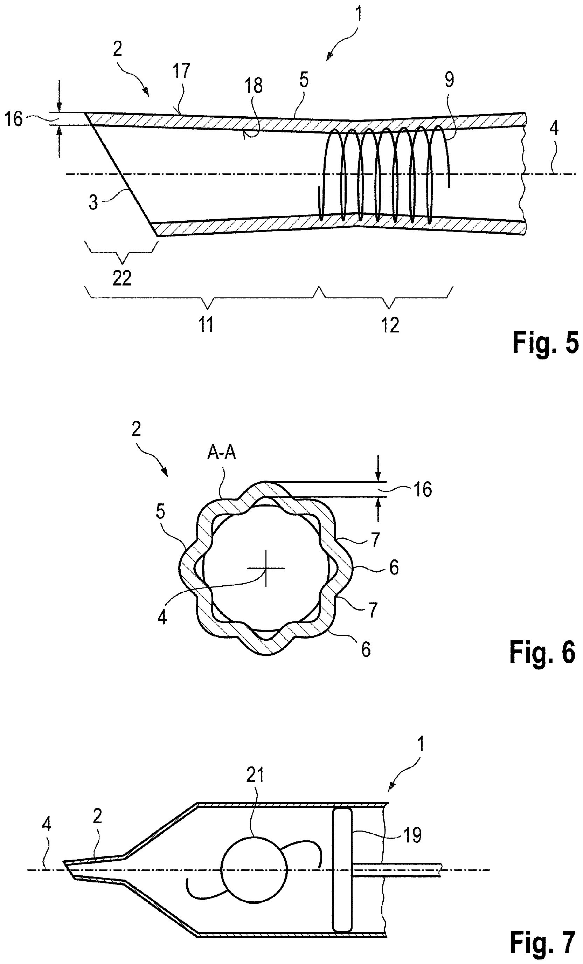

Wie es aus Figuren 1 bis 7 ersichtlich ist, weist ein Injektor 1 für ein Einführen einer Intraokularlinse 21 in den Kapselsack eines Auges eine Injektorachse 4, eine Injektorspitze 2, die eine Öffnung 3 und eine die Öffnung 3 begrenzende Wand 5 aufweist, und einen Kolben 19 auf, der in Richtung der Injektorachse 4 längsverlagerbar in dem Injektor 1 gelagert ist und eingerichtet ist, die Intraokularlinse 21 via die Öffnung 3 aus dem Injektor 1 zu befördern, wobei die Wand 5 in einem ersten Abschnitt 11, der von dem Ende 14 der Injektorspitze 2, an dem die Öffnung 3 angeordnet ist, ausgeht und sich in Richtung der Injektorachse 4 erstreckt, einen Elastizitätsmodul unter einer Zugbelastung in einem Bereich von 10 N/mm² bis 500 N/mm² hat und gewellt ausgeführt ist, so dass die Wand 5 in einer Querschnittsebene 20, deren Normale parallel zu der Injektorachse 4 angeordnet ist, eine Mehrzahl von Bergen 6 und eine Mehrzahl von Tälern 7 aufweist, wobei die Berge 6 und die Täler 7 in Umfangsrichtung 10 bezüglich der Injektorachse 4 abwechselnd angeordnet sind. Figur 1 zeigt, dass die Täler 7 in Richtung von der Öffnung 3 weg kontinuierlich immer flacher ausgebildet sein können. Figur 7 zeigt, dass der Injektor 1 die Intraokularlinse 21 aufweisen kann.

Figuren 2 bis 5 zeigen, dass die Injektorspitze 2 in dem Bereich des Endes 14 abgeschrägt sein kann. Beispielsweise kann die Wand 5 eine Stirnfläche 15 haben, die unmittelbar an die Öffnung 3 angrenzt und deren Normale einen Winkel mit der Injektorachse 4 einschließt, der von 0° verschieden ist und der beispielsweise von 20° bis 70° oder von 40° bis 50° beträgt. In dem Fall, dass die Injektorspitze 2 abgeschrägt ist, kann die Querschnittsebene 20 abseits desjenigen Bereichs 22, in dem die Injektorspitze 2 abgeschrägt ist, angeordnet sein. Figur 6 zeigt, dass die Wand 5 in der Querschnittsebene 20 und in Umfangsrichtung 10 bezüglich der Injektorachse 4 ohne Unterbrechungen ausgeführt sein kann. Zudem ist ersichtlich, dass die Wand 5 in der Querschnittsebene 20 und in Umfangsrichtung 10 bezüglich der Injektorachse 4 eine konstante Dicke 16 haben kann. Es ist zudem denkbar, dass die Dicke 16 in dem vollständigen ersten Abschnitt 11 konstant sein kann. Figur 6 zeigt zudem, dass die Wand 5 in der Querschnittsebene 20 und somit normal zur Injektorachse 4 punktsymmetrisch ausgeführt sein kann.

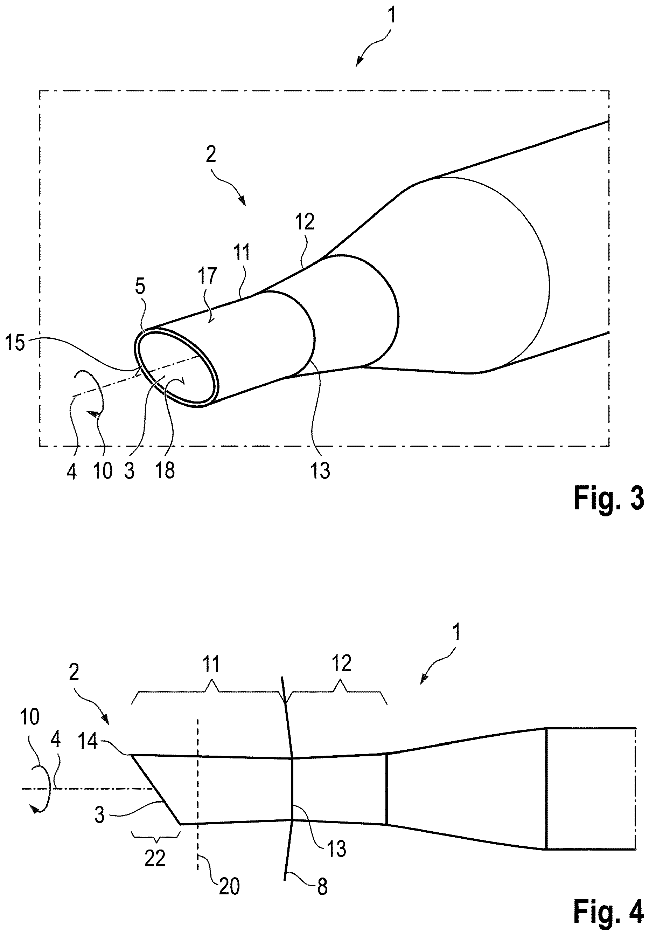

Insbesondere aus Figuren 1 und 3 ist ersichtlich, dass die Öffnung 3 vollständig in einer Öffnungsebene, die parallel zu der Querschnittsebene angeordnet sein kann oder deren Normale mit der Normalen der Querschnittsebene einen von 0° verschiedenen Winkel einschließen kann, liegen kann und dass die Wand 5 in dem ersten Abschnitt 11 bis auf die Öffnung 3 keine weiteren Aussparungen aufweisen kann. Zudem kann die Stirnfläche 15 vollständig in der Öffnungsebene liegen.

Die Wand 5 kann jeweils acht der Berge 6 und der Täler 7 aufweisen, wie es beispielsweise aus Figuren 1 und 6 ersichtlich ist. Es ist auch denkbar, dass die Wand 5 jeweils von vier bis zwölf der Berge 6 und der Täler 7 aufweist, insbesondere jeweils von sechs bis zehn der Berge 6 und der Täler 7.

Die Injektorspitze 4 kann einen entspannten Zustand, der in Figuren 1, 2 und 6 dargestellt ist und in dem die Intraokularlinse nicht in der Injektorspitze 4 angeordnet ist, einen teilweise gespannten Zustand, in dem die Wand 5 in dem ersten Abschnitt 11 durch ein Einführen der Intraokularlinse in die Injektorspitze 2 unter eine mechanische Spannung gesetzt ist, und einen maximal gespannten Zustand aufweisen, der in Figuren 3 bis 5 dargestellt ist und in dem die Wand 5 durch das Einführen der Intraokularlinse maximal gespannt ist, wobei die Öffnung 3 in dem maximal gespannten Zustand größer als in dem entspannten Zustand ist. Der besseren Übersicht halber ist eine die mechanische Spannung des ersten Abschnittes 11 verursachende Intraokularlinse 21 nicht eingezeichnet. Die Wand 5 kann eingerichtet sein, dass die Injektorspitze 2 wieder in den in Figuren 1, 2 und 6 dargestellten entspannten Zustand zurückkehrt, nachdem die Intraokularlinse 21 den Injektor 1 verlassen hat.

Figur 2 zeigt, dass in dem ersten Abschnitt 11 die Injektorspitze 2 in Richtung zu der Öffnung 3 hin ihren Querschnitt, dessen Normale parallel zu der Injektorachse 4 angeordnet ist, in dem entspannten Zustand verkleinern kann.

Figur 4 zeigt, dass in dem ersten Abschnitt 11 die Injektorspitze 2 in Richtung zu der Öffnung 3 hin ihren Querschnitt, dessen Normale parallel zu der Injektorachse 4 angeordnet ist, in dem maximal gespannten Zustand vergrößern kann. Alternativ ist denkbar, dass in dem ersten Abschnitt 11 die Injektorspitze 2 in Richtung zu der Öffnung 3 hin ihren Querschnitt, dessen Normale parallel zu der Injektorachse 4 angeordnet ist, in dem maximal gespannten Zustand gleich groß belassen kann. Dabei kann jeweils der Querschnitt an der Innenseite 18 der Wand 5 und/oder an der Außenseite 17 der Wand gemeint sein. In dem ersten Abschnitt 11 kann die Wand 5 an deren Außenseite 17 und/oder deren Innenseite 18 in der Querschnittsebene 20 und in dem maximal gespannten Zustand beispielsweise eine kreisförmige, elliptische und/oder ovale Form haben.

Wie es aus Figuren 1 bis 5 ersichtlich ist, kann die Wand 5 einen zweiten Abschnitt 12 aufweisen, der sich in Richtung der Injektorachse 4 erstreckt und in dem die Wand 5 ein Elastizitätsmodul unter der Zugbelastung von höher als 900 N/mm² hat und nicht gewellt ist. Der zweite Abschnitt 12 kann dabei unmittelbar an den ersten Abschnitt 11 angrenzen, wie es auch die Figuren 1 bis 5 zeigen. Dabei ist denkbar, dass die Wand 5 in dem gesamten ersten Abschnitt 1 einen Elastizitätsmodul unter der Zugbelastung im Bereich von 10 N/mm² bis 500 N/mm² hat. Zudem ist denkbar, dass die Wand 5 in dem gesamten ersten Abschnitt 11 gewellt ausgeführt ist. Alternativ ist denkbar, dass die Wand 5 lediglich in einem Bereich, der unmittelbar an den zweiten Abschnitt 12 angrenzt, nicht gewellt ausgeführt ist, wie es beispielsweise aus Figur 1 ersichtlich ist.

Der zweite Abschnitt 12 und/oder eine Grenze zwischen dem ersten Abschnitt 11 und dem zweiten Abschnitt 12 sind dazu vorgesehen, eine Hornhaut 8 des Auges zu kontaktieren, wenn die Injektorspitze 2 in das Auge eingeführt wird (vergleiche Figuren 2 und 4). Die Injektorspitze 2 kann eine Markierung 13 aufweisen, um die Grenze zwischen dem ersten Abschnitt 11 und dem zweiten Abschnitt 12 zu kennzeichnen (siehe Figuren 1 bis 4) .

Die Wand 5 in dem zweiten Abschnitt 12 kann somit einen Elastizitätsmodul unter der Zugbelastung von höher als 900 N/mm² haben. Alternativ dazu kann die Dicke 16 der Wand 5 in dem zweiten Abschnitt 12 größer als in dem ersten Abschnitt sein, wobei der Elastizitätsmodul jenem des ersten Abschnitts entspricht. Alternativ oder zusätzlich kann der zweite Abschnitt 12 ein Verstärkungsstück aufweisen, das in der Wand 5 angeordnet ist. Figur 5, in der die Injektorspitze 2 im Längsschnitt und das Verstärkungsstück vollständig dargestellt ist, zeigt, dass das Verstärkungsstück eine Helix 9 sein kann. Die Steigung der Helix 9 kann dabei entlang der Injektorachse 4 variieren. Alternativ ist denkbar, dass das Verstärkungsstück die Helix 9 aufweist, ein zylindermantelförmiges Teil ist oder aufweist und/oder ein kegelmantelförmiges Teil ist oder aufweist. Die Helix 9 hat den Vorteil, dass ein Bediener in dem Fall, dass die Wand 5 transparent ist, in die Injektorspitze 2 hinein sehen kann und ein Verlagern der Intraokularlinse 21 kontrollieren kann. Das zylindermantelförmige Teil und das kegelmantelförmige Teil haben den Vorteil, dass sie den zweiten Abschnitt besonders gut versteifen können. Das Verstärkungsstück kann ein Metall oder eine Legierung aufweisen oder aus dem Metall oder der Legierung bestehen. Figur 5 zeigt zudem, dass die Dicke 16 der Wand 5 in dem ersten Abschnitt 11 gleich der Dicke der Wand 5 in dem zweiten Abschnitt 12 sein kann.

Die Injektorspitze 2 mit dem Verstärkungsstück kann beispielsweise durch ein Spritzgussverfahren hergestellt werden. Alternativ ist denkbar, dass die Injektorspitze 2 mit dem Verstärkungsstück hergestellt wird, indem ein Rohr aus einem polymeren Werkstoff extrudiert wird, auf das Rohr das Verstärkungsstück aufgebracht wird, insbesondere die Spirale aufgewickelt wird, und danach eine äußere Schicht aus einem polymeren Werkstoff auf das Rohr und das Verstärkungsstück aufgebracht wird. Zudem kann vor dem Aufbringen des Verstärkungsstücks eine Klebeschicht außen auf das Rohr aufgebracht werden.

### Bezugszeichenliste

- 1: Injektor
- 2: Injektorspitze
- 3: Öffnung
- 4: Injektorachse
- 5: Wand
- 6: Berg
- 7: Tal
- 8: Hornhaut
- 9: Helix
- 10: Umfangsrichtung
- 11: erster Abschnitt
- 12: zweiter Abschnitt
- 13: Markierung
- 14: Ende
- 15: Stirnfläche
- 16: Dicke
- 17: Außenseite
- 18: Innenseite
- 19: Kolben
- 20: Querschnittsebene
- 21: Intraokularlinse
- 22: abgeschrägter Bereich

## Patentansprüche

1. Injektor für ein Einführen einer Intraokularlinse in den Kapselsack eines Auges, mit einer Injektorachse (4), einer Injektorspitze (2), die eine Öffnung (3) und eine die Öffnung (3) begrenzende Wand (5) aufweist, einem Kolben (19), der in Richtung der Injektorachse (4) längsverlagerbar in dem Injektor (1) gelagert ist und eingerichtet ist, die Intraokularlinse via die Öffnung (3) aus dem Injektor (1) zu befördern, wobei die Wand (5) in einem ersten Abschnitt (11), der von dem Ende (14) der Injektorspitze (2), an dem die Öffnung (3) angeordnet ist, ausgeht und sich in Richtung der Injektorachse (4) erstreckt, einen Elastizitätsmodul unter einer Zugbelastung in einem Bereich von 10 N/mm² bis 500 N/mm² hat und gewellt ausgeführt ist, so dass die Wand (5) in einer Querschnittsebene (20), deren Normale parallel zu der Injektorachse (4) angeordnet ist, eine Mehrzahl von Bergen (6) und eine Mehrzahl von Tälern (7) aufweist, wobei die Berge (6) und die Täler (7) in Umfangsrichtung (10) bezüglich der Injektorachse (4) abwechselnd angeordnet sind, **dadurch gekennzeichnet, dass** die Injektorspitze (2) in dem Bereich des Endes (14) abgeschrägt ist, indem die Wand (5) eine Stirnfläche (15) hat, die unmittelbar an die Öffnung (3) angrenzt und deren Normale einen Winkel mit der Injektorachse (4) einschließt, der von 0° verschieden ist.

2. Injektor gemäß Anspruch 1, wobei die Injektorspitze (4) einen entspannten Zustand, in dem die Intraokularlinse nicht in der Injektorspitze (4) angeordnet ist, einen teilweise gespannten Zustand, in dem die Wand (5) in dem ersten Abschnitt (11) durch ein Einführen der Intraokularlinse in die Injektorspitze (2) unter eine mechanische Spannung gesetzt ist, und einen maximal gespannten Zustand aufweist, in dem die Wand (5) durch das Einführen der Intraokularlinse maximal gespannt ist, wobei die Öffnung (3) in dem maximal gespannten Zustand größer als in dem entspannten Zustand ist.

3. Injektor gemäß Anspruch 2, wobei in dem ersten Abschnitt (11) die Injektorspitze (2) in Richtung zu der Öffnung (3) hin ihren Querschnitt, dessen Normale parallel zu der Injektorachse (4) angeordnet ist, in dem entspannten Zustand verkleinert.

4. Injektor gemäß Anspruch 2 oder 3, wobei in dem ersten Abschnitt (11) die Injektorspitze (2) in Richtung zu der Öffnung (3) hin ihren Querschnitt, dessen Normale parallel zu der Injektorachse (4) angeordnet ist, in dem maximal gespannten Zustand vergrößert oder gleich groß belässt.

5. Injektor gemäß einem der Ansprüche 2 bis 4, wobei in dem ersten Abschnitt (11) die Wand (5) an deren Außenseite (17) und/oder deren Innenseite (18) in der Querschnittsebene (20) und in dem maximal gespannten Zustand eine kreisförmige, elliptische und/oder ovale Form hat.

6. Injektor gemäß einem der Ansprüche 1 bis 5, wobei die Wand (5) in Umfangsrichtung (10) bezüglich der Injektorachse (4) eine konstante Dicke (16) hat.

7. Injektor gemäß einem der Ansprüche 1 bis 6, wobei die Wand (5) einen zweiten Abschnitt (12) aufweist, der sich in Richtung der Injektorachse (4) erstreckt und in dem die Wand (5) einen Elastizitätsmodul unter der Zugbelastung von höher als 900 N/mm² hat.

8. Injektor gemäß Anspruch 7, wobei der zweite Abschnitt (12) ein Verstärkungsstück aufweist, das in der Wand (5) angeordnet ist, wobei das Verstärkungsstück eine Helix (9), ein zylindermantelförmiges Teil und/oder einen kegelmantelförmiges Teil ist oder aufweist.

9. Injektor gemäß einem der Ansprüche 1 bis 8, wobei die Wand (5) jeweils von vier bis zwölf der Berge (6) und der Täler (7) aufweist, insbesondere jeweils von sechs bis zehn der Berge (6) und der Täler (7) oder jeweils acht der Berge (6) und der Täler (7) aufweist.

## Claims

1. Injector for introducing an intraocular lens into the capsular bag of an eye, having an injector axis (4), an injector tip (2) which has an opening (3) and a wall (5) that delimits the opening (3), a piston (19) which is mounted so as to be longitudinally displaceable in the direction of the injector axis (4) in the injector (1) and is designed to convey the intraocular lens out of the injector (1) via the opening (3), wherein the wall (5), in a first portion (11) that proceeds from the end (14) of the injector tip (2) where the opening (3) is disposed and extends in the direction of the injector axis (4), has a modulus of elasticity under a tensile load in a range of 10 N/mm² to 500 N/mm² and is corrugated so that the wall (5) in a cross-sectional plane (20), the normal of which is disposed parallel to the injector axis (4), has a plurality of peaks (6) and a plurality of troughs (7), wherein the peaks (6) and the troughs (7) are disposed alternately in the circumferential direction (10) with respect to the injector axis (4), **characterized in that** the injector tip (2) is bevelled in the region of the end (14) by the wall (5) having an end face (15) which is directly adjacent to the opening (3), the normal of said end face (15) enclosing with the injector axis (4) an angle which is different from 0°.

2. Injector according to Claim 1, wherein the injector tip (4) has a relaxed state in which the intraocular lens is not disposed in the injector tip (4), a partially tensioned state in which the wall (5) in the first portion (11) is subjected to mechanical tension by introducing the intraocular lens into the injector tip (2), and a maximum tensioned state in which the wall (5) is tensioned to the maximum by the introduction of the intraocular lens, wherein the opening (3) is larger in the maximum tensioned state than in the relaxed state.

3. Injector according to Claim 2, wherein in the first portion (11) the injector tip (2) in terms of its cross section, the normal of which is disposed parallel to the injector axis (4), decreases in the direction towards the opening (3) in the relaxed state.

4. Injector according to Claim 2 or 3, wherein in the first portion (11) the injector tip (2) in terms of its cross section, the normal of which is disposed parallel to the injector axis (4), increases or remains the same in the direction towards the opening (3) in the maximum tensioned state.

5. Injector according to one of Claims 2 to 4, wherein in the first portion (11) the wall (5) on its outside (17) and/or its inside (18) in the cross-sectional plane (20) and in the maximum tensioned state has a circular, elliptical and/or oval shape.

6. Injector according to one of Claims 1 to 5, wherein the wall (5) in the circumferential direction (10) with respect to the injector axis (4) has a constant thickness (16) .

7. Injector according to one of Claims 1 to 6, wherein the wall (5) has a second portion (12), which extends in the direction of the injector axis (4) and in which the wall (5) has a modulus of elasticity under the tensile load of more than 900 N/mm².

8. Injector according to Claim 7, wherein the second portion (12) has a reinforcing piece which is disposed in the wall (5), wherein the reinforcing piece is or has a helix (9), a cylindrical shell-shaped part and/or a conical shell-shaped part.

9. Injector according to one of Claims 1 to 8, wherein the wall (5) has in each case four to twelve of the peaks (6) and of the troughs (7), in particular in each case six to ten of the peaks (6) and of the troughs (7), or in each case eight of the peaks (6) and of the troughs (7) .

## Revendications

1. Injecteur pour une introduction d'une lentille intraoculaire dans le sac capsulaire d'un œil, avec un axe d'injecteur (4), une pointe d'injecteur (2) qui présente une ouverture (3) et une paroi (5) délimitant l'ouverture (3), un piston (19) qui est logé dans l'injecteur (1) de manière à pouvoir se déplacer longitudinalement dans la direction de l'axe d'injecteur (4) et qui est conçu de façon à transporter la lentille intraoculaire hors de l'injecteur (1) via l'ouverture (3), la paroi (5) étant située dans une première portion (11) qui part de l'extrémité (14) de la pointe d'injecteur (2) où est située l'ouverture (3) et qui s'étend dans la direction de l'axe d'injecteur (4), qui a un module d'élasticité sous une charge de traction située dans une gamme allant de 10 N/mm² à 500 N/mm² et est réalisée de manière ondulée, de sorte que la paroi (5) présente, dans un plan de section transversale (20) dont la normale est disposée parallèlement à l'axe d'injecteur (4), une pluralité de crêtes (6) et une pluralité de vallées (7), les crêtes (6) et les vallées (7) étant disposées en alternance dans la direction circonférentielle (10) par rapport à l'axe (4) de l'injecteur, **caractérisé en ce que** la pointe d'injecteur (2) est biseautée dans la zone de l'extrémité (14), la paroi (5) ayant une face frontale (15) qui est directement adjacente à l'ouverture (3) et dont la normale forme un angle avec l'axe (4) de l'injecteur qui est différent de 0°.

2. Injecteur selon la revendication 1, dans lequel la pointe d'injecteur (4) présente un état détendu dans lequel la lentille intraoculaire n'est pas disposée dans la pointe d'injecteur (4), un état partiellement tendu dans lequel la paroi (5) est mise sous tension mécanique dans la première portion (11) par une introduction de la lentille intraoculaire dans la pointe d'injecteur (2), et un état de tension maximale dans lequel la paroi (5) est mise sous tension maximale par l'introduction de la lentille intraoculaire, l'ouverture (3) étant plus grande dans l'état de tension maximale que dans l'état détendu.

3. Injecteur selon la revendication 2, dans lequel, dans la première portion (11), la pointe d'injecteur (2) a sa section transversale, dont la normale est disposée parallèlement à l'axe d'injecteur (4), qui diminue en direction de l'ouverture (3) à l'état détendu.

4. Injecteur selon la revendication 2 ou la revendication 3, dans lequel, dans la première portion (11), la pointe d'injecteur (2) a sa section transversale, dont la normale est disposée parallèlement à l'axe d'injecteur (4), qui augmente en direction de l'ouverture (3) ou a cette section restant égale dans l'état de tension maximale.

5. Injecteur selon l'une des revendications 2 à 4, dans lequel, dans la première portion (11), la paroi (5) a une forme circulaire, elliptique et/ou ovale sur sa face externe (17) et/ou sa face interne (18) dans le plan de la section (20) et à l'état de tension maximale.

6. Injecteur selon l'une des revendications 1 à 5, dans lequel la paroi (5) a une épaisseur (16) constante dans la direction circonférentielle (10) par rapport à l'axe (4) de l'injecteur.

7. Injecteur selon l'une des revendications 1 à 6, dans lequel la paroi (5) comprend une deuxième portion (12) qui s'étend dans la direction de l'axe (4) de l'injecteur et dans laquelle la paroi (5) a un module d'élasticité sous une charge de traction supérieur à 900 N/mm².

8. Injecteur selon la revendication 7, dans lequel la deuxième portion (12) comprend une pièce de renfort disposée dans la paroi (5), la pièce de renfort étant ou comprenant une hélice (9), une pièce en forme d'enveloppe cylindrique et/ou une pièce en forme d'enveloppe conique.

9. Injecteur selon l'une des revendications 1 à 8, dans lequel la paroi (5) comporte respectivement de quatre à douze crêtes (6) et vallées (7), notamment respectivement de six à dix crêtes (6) et vallées (7) ou respectivement huit crêtes (6) et vallées (7).
